# EUROPEAN PATENT APPLICATION

(11) **EP 3 181 154 A1**
(43) Date of publication of application: **21.06.2017**
(21) Application number: 15201179.7
(22) Date of filing: 18.12.2015
(51) Int. Cl.: A61L 15/26, A61L 15/32, A61L 15/40

(54) **WOUND CARE PRODUCT WITH ECM LAYER**

(71) Applicant: BSN medical GmbH, 20253 Hamburg (DE)
(72) Inventor: Lanfer, Babette, 20149 Hamburg (DE); Arshi, Annahit, 20357 Hamburg (DE); Hemmrich, Karsten, 40589 Düsseldorf (DE); Wilhelms, Tim, 22041 Hamburg (DE); Blumenthal, Nils, 22765 Hamburg (DE)
(74) Representative: Jostarndt Patentanwalts-AG

(57) **Abstract**

The invention relates to a multilayered wound care product comprising at least one extracellular matrix (ECM) layer and at least one polymer layer comprising collagen and at least one further biodegradable polymer. In particular, the invention relates to an improved wound care with an ECM layer attached to a layer of composite fibers of collagen and at least one further biodegradable polymer. The invention further relates to the use of said wound care product for treatment of skin wounds. The invention also relates to a method of producing said wound care product.

## Description

### Field of the invention:

The invention relates to a multilayered wound care product comprising an extracellular matrix (ECM) layer and a polymer layer comprising collagen and at least one further biodegradable polymer. In particular, the invention relates to an improved wound care with an ECM layer attached to a layer of composite fibers of collagen and at least one biodegradable polymer layer. The invention further relates to the use of said wound care product for treatment of skin wounds. The invention finally relates to a method of producing said wound care product.

### Background of the invention

Wound care products are used in wound healing, in particular of skin wounds. Typically, a wound care product such as a dressing includes a backing and a wound contacting layer and is placed on a wound in order to protect the wound from contaminations that might lead to infections.

Wound care products are also used to promote organization, growth and differentiation of cells required for the growth of new tissue in order to close a wound. In this respect, wound care products typically include a scaffold with a certain degree of porosity as a structure for the ingrowth of cells. After cell ingrowth, the scaffold may be difficult to remove. To circumvent this issue, often temporary scaffolds are provided which are biodegradable and may be broken down by surrounding tissues. As a consequence, no removal of the scaffold is required.

To further promote wound healing, the use of extracellular matrix (ECM) material is known. Moreover, the use of ECMs and ECM material as part of a wound care product is known. ECM is particularly useful in the field of wound healing because it contains growth factors and other biological molecules that promote cell growth and thus support wound healing. Wound care products containing ECM material are often in the form of sheets or hydrogels. Respective products provide cell growth promoting molecules as well as a scaffold for cell ingrowth. For example, the product Xelma of Mölnlycke (Erkrath, Germany) is a hydrogel which contains a protein extract from an extracellular matrix in the form of ECM particles. However, the ECM protein extract can mimic the original ECM with its wound-healing supporting properties only in a limited way. Furthermore, these hydrogelembedded ECM particles are not in intimate contact with the wound surface.

In EP 1 942 957 A1, a temporary composite scaffold is described that comprises embedded, discrete, cell free ECM particles. The product combines the physical properties of its temporary scaffold with the reconstructive properties of the ECM particles. Also here, the use of processed ECM particles lacks the biological functionality of an intact ECM sheet.

Examples of products in the form of sheets include OASIS from Healthpoint (lyophilized porcine ECM sheet) and Graftjacket from Wright Medical (lyophilized human ECM sheet). Both sheets provide a scaffold for wound healing. Due to the use of an intact ECM layer, these products are very cost intensive and thus are not broadly used in the clinical setting.

Hence, there is still a need for an improved wound care product providing the biological functionality of an ECM in a cost sensitive manner. The objective of the present invention thus is to provide a wound care product which overcomes at least one of the above mentioned disadvantages.

This problem is solved by provision of a wound care product according to claim 1. Specific embodiments are subject matter of further independent claims.

### Summary of the invention:

In a first aspect the present invention provides a multilayered wound care product comprising:
(a) at least one extracellular matrix (ECM) layer;
(b) at least one polymer layer comprising collagen and at least one further biodegradable polymer; and optionally
(c) a covering layer.

The wound care product of the present invention has several advantages over wound care products known in the prior art.

By adding a polymer layer comprising collagen and at least one further biodegradable polymer to the ECM layer, it is possible to combine the physical properties (e.g. strength, softness, flexibility, biodegradation) of the polymer layer with the reconstructive properties of the ECM.

The collagen of the polymer layer supports the function of the ECM layer by offering a substrate for matrix metalloproteases (MMPs) which otherwise could impair the wound healing process.

Furthermore, as a biodegradable material, the collagen can be degraded by the organism simultaneously to the reestablishment of the tissue in the wound. This represents an important clinical advantage, since the wound care product has not to be removed from the wound after or during the healing process.

Due to the presence of the further biodegradable polymer, the amount of the utilized collagen can be reduced while retaining the biodegradable properties of the layer.

Since the polymer layer is preferably located on top of the ECM layer and is therefore not in direct contact with the wound, there is no formation of "jelly-like collagen islands" which can be observed for collagen-based products such as Promogran Matrix (Systagenix, Gatwick, UK).

Also here, the broad spectrum of further biodegradable polymers allows the selection of the optimal material with regard to costs, biodegradability, strength and flexibility.

Due to the presence of a stabilizing polymer layer, the thickness of the ECM matrix can be markedly reduced, allowing the use of ECM layers which can be generated by preparing thin ECM tissue sections.

Since these thin ECM slices still represent intact ECM sheets, there is no loss of biological activity as compared to further processed (particulate) ECM material.

Due to the reduction in ECM material, the costs for the wound care product can considerably be reduced while the full biological ECM activity is retained.

Since the present invention combines important demands like full ECM functionality and enhanced initial stability with a complete biodegradability of the product within one multilayer, further layers are not necessary.

However, the multilayer of the invention can be provided with additional layers such as, e.g., a covering layer.

By an individual selection of the utilized ECM material, the collagen, the further biodegradable polymer and the layer structures and thicknesses, the wound care product can easily be adapted to the specific requirements of the underlying injury. Hence, it offers an enormous flexibility of application.

Since the wound care product of the invention is based on known components, it can be easily produced in a cost efficient manner.

### Detailed description of the invention

The inventive wound care product may be used as such, as a dressing or in combination with a backing known in the art. For example, the wound care product according to the present invention may be temporarily adhered to a backing. This backing may be removed from the wound care product once applied to the wound or after cellular ingrowth has started.

The extracellular matrix (ECM), as understood in the context of the present application, is the non-cellular portion or material of animal or human tissues that surrounds cells. The ECM material may be obtained from any kind of tissue by the use of physical, enzymatic, and/or chemical methods well known in the art. In principal, the major components of ECM comprise fibrous elements (particularly collagen, chitosan, elastin or reticulin), link proteins (for example fibronectin or laminin) and space-filling molecules (for example glycosaminoglycans). Moreover, ECM typically includes growth factors, cytokines, and other biological molecules that are known to attract cells and to promote cellular proliferation.

According to the invention the ECM layer consists of a particulate ECM or sheet-like ECM.

In a preferred embodiment of the invention a sheet-like ECM (herein synonymous with the term "ECM sheet") is used which is preferably a decellularized ECM sheet. Hereby, the isolated tissue is treated with a series of oxidizing agents that remove the cellular and nuclear material from the isolated tissue while substantially retaining the biological and mechanical properties, as well as the biochemical composition of the resulting ECM.

Decellularizing agents that can be used in the methods of the invention are those agents that are effective in removing cellular and nuclear material from the isolated tissue without substantially compromising the biocompatible, biological and mechanical properties or the biochemical composition of the ECM. Examples of decellularizing agents that can be used for decellularization of the tissue include, but are not limited to, oxidizing agents (e.g., hydrogen peroxide, peroxy acids), ascorbic acid (vitamin C), chelating agents (e.g., EDTA, EGTA), methionine, cysteine, maleic acid, and polymers that bind to DNA (e.g., Poly-L-lysine, polyethylimine (PEI) and polyamidoamine (PAMAM)).

In a preferred embodiment of the invention the ECM sheet is obtained from tissue and cut along the layer plane to yield an ECM sheet with a reduced thickness.

In one embodiment of the invention the polymer layer is attached with a film that controls water steam permeation and/or to control the fluid handling capacity. In a preferred embodiment this film is a polyurethane film. The additional layer is useful to develop a wound care product that is useful for different exuding levels.

In a further preferred embodiment of the invention, the ECM sheet has a thickness between 50 and 250 µm, preferably between 75 and 150 µm and more preferably between 80 and 120 µm. Due to the presence of the further collagen/polymer layer, the ECM sheet can have a reduced thickness. A thickness of the ECM sheet of approximately 100 µm has been found to be particularly preferred because it can still be produced by conventionally cutting devices and shows biological ECM-functionality as well as favorable degradation rates.

However, such an embodiment may also, for example, be obtained by coating the polymer layer with a powder of ECM material.

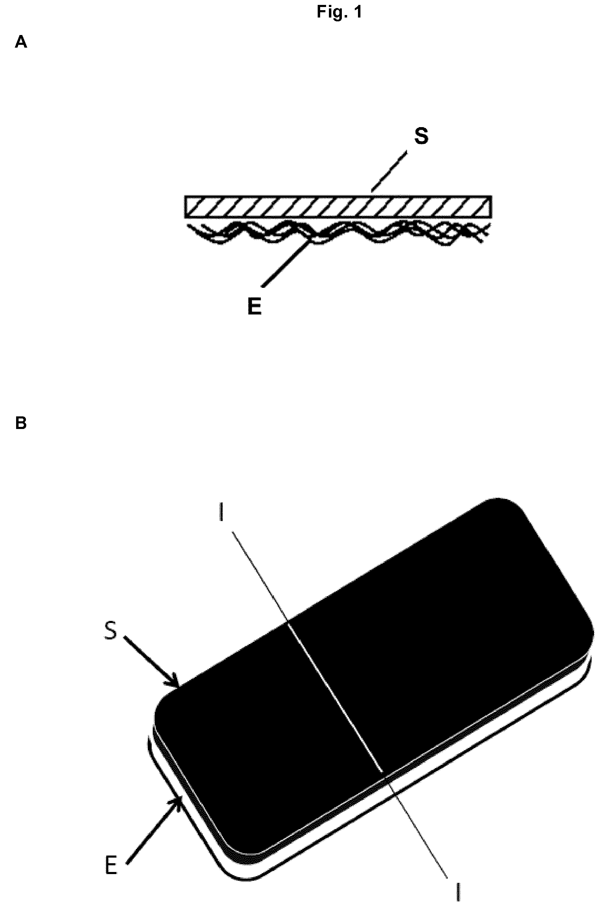

The dimension of the surface area of the wound care product may be selected according to the size of the wound. Typically, a wound care product of the present invention may have a surface area of from surface area of from 5 cm² to 400 cm², and particularly of from 25 cm² to 200 cm², such as 100 cm². Also the shape of the wound care product according to the present invention may vary with respect to the wound to be treated, and the present invention encompasses, for example, rectangular, square-, circle- or oval-shaped wound care products. For example, the wound care product may have a rectangular shape with rounded corners as depicted in Fig. 1B.

For preparing the ECM layer the skilled person can draw on a broad spectrum of potential sources. In one embodiment the ECM layer can be isolated from *in vitro* cultivated cells. Preferably cells include cultured chondrocytes, fibroblasts, keratinocytes or epithelial cells.

The ECM material used in the present invention may be obtained from tissue harvested from any animal, but in particular from animals raised for meat production, including but not limited to, pigs, cattle and sheep. Using these animals as sources for the ECM material reduces the costs of the material. Other warm-blooded vertebrates may also hold as a source of tissue. Moreover, the ECM material may also be obtained from tissue of human corpses. The ECM material can be obtained from any organ, but is preferably obtained from intestinal tissue, bladders, liver, spleen, stomach, lymph nodes or skin. Preferably, the ECM material used in the present invention is derived from human cadaver skin, porcine urinary bladder submucosa (UBS), porcine urinary bladder matrix (UBM), or porcine small intestinal submucosa (SIS). When human tissue is used as the source of the ECM material, the risk of an immunogenic response or immune rejection can be reduced. However, using ECM material of animal origin may reduce the risk of a transfer of diseases. In a preferred embodiment of the present invention, the ECM material is of animal origin. Particularly preferred is an ECM material derived from warm-blooded mammals, due to their similarity to humans. Preferably, the ECM material used in the present invention is of porcine origin. Most preferred is an ECM material derived from Urinary Bladder Matrix (UBM) or small intestine submucosa (SIS) of porcine origin.

The wound care product of the present invention contains at least two layers, a polymer layer and a separate and distinct ECM layer.

Typically, for obtaining a wound care product according to the present invention, an ECM coating has been applied to the polymer layer in the form of a sheet of ECM material or in the form of a powder of ECM material. Thus, in a preferred embodiment the wound care product may also be referred to as comprising a polymer layer with a powdered ECM coating.

In a particularly preferred embodiment, the polymer layer is coated on both sides with an ECM coating. If the ECM material has been applied to the polymer layer in the form of a powder, such an embodiment may also be referred to as a wound care product comprising a polymer layer powdered on either side with an ECM coating.

Preferred ECM materials for use in the present invention contain further bioactive molecules which might be already present in the tissue source or alternatively added to the ECM material after preparation. For example, the ECM material may contain at least one compound from a group including fibroblast growth factor-2 (basic FGF), transforming growth factor-beta (TGF-beta) platelet derived growth factor (PDGF), cartilage derived growth factor (CDGF), epidermal growth factor (EGF), transforming growth factor alpha (TGF-alpha), thrombospondin, osteopontin, angiotensin converting enzyme (ACE), bone morphogenetic protein (BMP), NGF (nerve growth factor), tumor necrosis factor alpha (TNF-α), acidic fibroblast growth factor (aFGF), hepatocyte growth factor (HGF), insulin-like growth factors 1 and 2 (IGF-1 and IGF-2), stromal derived factor 1 alpha (SDF-1 alpha), ciliary neurotrophic factor (CNTF), neurotrophin-3, neurotrophin-4, neurotrophin-5, pleiotrophin protein (neurite growth-promoting factor 1), midkine protein (neurite growth-promoting factor 2), brain-derived neurotrophic factor (BDNF), tumor angiogenesis factor (TAF), corticotrophin releasing factor (CRF), interleukin-8 (IL-8), granulocyte-macrophage colony stimulating factor (GM-CSF), and/or vascular endothelial growth factor (VEGF). Further preferred is that the ECM material used in the present invention contains additional bioactive components including, for example, one or more of collagens, glycosaminoglycans, glycoproteins and proteoglycans. The ECM material may further include the basement membrane or parts thereof, which is typically made up mostly of type IV collagen, laminins and proteoglycans.

The at least one polymer layer of the invention is preferably directly attached to the at least one ECM layer.

The skilled person can choose from a great variety of adhesive agents for connecting these two layers. In a preferred embodiment the at least one polymer layer and the at least one ECM layer are connected by using a gelatin glue, or chemical crosslinking by e.g. glutaraldehyde, or derivatization of carboxylic groups with N-hydroxysuccinimide (NHS) and further reaction of the NHS-activated acid by use of a coupling reagent such as dicyclohexylcarbodiimide (DCC) or ethyl(dimethylaminopropyl) carbodiimide (EDC). Hereby the use of gelatin glue is most preferred while representing a biocompatible adhesive agent.

In a further embodiment, the polymer layer and the ECM layer may be separated by an additional intermediate layer that is located between both layers. According to this embodiment of the wound care product, there is no direct contact between the polymer layer and the ECM layer. The advantage of this embodiment could be an enhanced strength of the multilayer or a control of the passage of cells or other biological molecules between the two layers. Further, this additional intermediate layer may be used to ensure the attachment of both layers to the product. The additional intermediate layer (IL) may comprise, for example, is designed and composed according to prior art, and may comprise, for example, a material based on polysaccharide, animal- or plant protein, animal connective tissue, fibrin, silicone, epoxide, acrylate, cyano-acrylate, (poly)urethane, enzymes (such as transglutaminase), L-DOPA or also gelatin-resorcinol-formaldehyde-glutaraldehyde ("French glue"), and may have a thickness of from 1 µm to 500 µm, and particularly of from 3 µm to 150 µm. It shall be understood that such an additional intermediate layer or separating layer may be provided between any layers in any of the embodiments described herein, even if not explicitly mentioned in the context of the other embodiments.

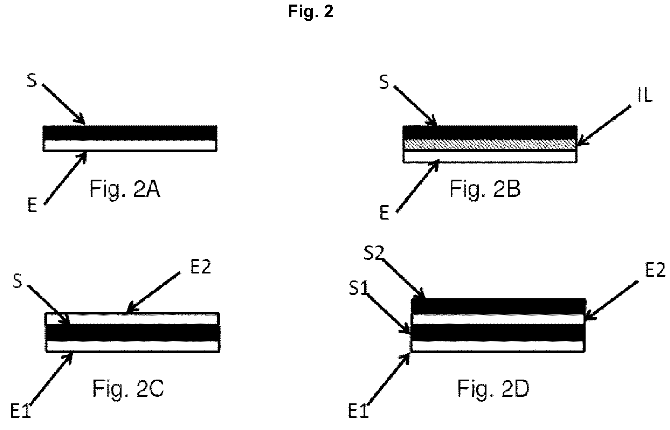

It shall be understood that the wound care product according to the present invention may include still further layers of ECM material and further polymer layers. Preferably, the polymer and ECM layers in such embodiments are alternating, as depicted, for example, in Fig. 2D. Therein, two ECM coatings or layers (E1 and E2) have been applied to a first polymer layer (S1), and a further polymer layer (S2) has been additionally applied to the second ECM coating (E2). Accordingly, the wound care product of this embodiment includes alternating polymer and ECM layers.

In one embodiment the multilayer of the invention can be provided with additional layers such as, e.g., a covering layer.

In a preferred embodiment the covering layer is a polyurethane film having preferably a thickness between 10 and 50 µm, preferably between 15 and 40 µm and more preferably between 18 and 30 µm.

In a further embodiment, the ECM layer completely encapsulates the polymer layer. Such an arrangement of the polymer layer and ECM layer brings about the advantage of a ECM that completely surrounds the supportive polymer layer which can be used e.g. for complex wounds or wounds within the body.

The wound care product of the invention comprising three layers may be adapted to include even further layers (e.g. a further ECM layer completely encapsulating the polymer layer. Several of such further layers may be provided as alternating layers of polymer and ECM layers.

In one embodiment of the invention the at least one polymer layer is a polymeric film, thus representing a thin sheet structure which preferably has pores enabling the passage of water vapor and oxygen but preventing the passage of microorganisms.

In an alternative and preferred embodiment the at least one polymer layer is a 3D-scaffold comprising open pores or at least a porosity which enables cells to enter and grow within the scaffold. Porosity is defined as P=1-p (V/M) wherein P is the scaffold porosity, ρ is the density of the polymeric system used, M is the weight and V is the volume of the fabricated scaffolds.

The polymer layer of the invention, when formed as a 3D-scaffold, preferentially has a porosity of more than 50%, more preferably of more than 80%, even more preferably of more than 90% and especially preferably of more than 95%.

In an even more preferred embodiment the polymer layer of the invention is a hydrogel. In general, hydrogels comprise three-dimensional (3D) crosslinked networks of hydrophilic polymers that swell in water. Water can penetrate in between the polymer chains of the polymer network, subsequently causing swelling and the formation of a hydrogel. Hydrogels are superabsorbent (e.g. they can contain over 99% water) and possess a degree of flexibility which is, due to their significant water content, very similar to natural tissue.

In another embodiment of the invention the polymer layer represents a mixture of fibers made from on or more biodegradable polymers and collagen fibers. Said fiber mixture can be provided as a loose mesh of fibers, as a fleece, a non-woven or a woven.

A preferred period of time, in which the further biodegradable polymer is completely degraded after the wound care product has been applied to the wound, is in the range of 1 day to 10 weeks, depending, for example, on the actual embodiment of the wound care product used, the thickness or composition of the polymer layer, as well as on the particularities of the wound to be treated. For example, when a wound care product according to the present invention is applied to an open wound, the preferred period for the temporary scaffold to vanish is 1 to 10 days, more preferably 2 to 7 days.

In one embodiment of the invention the further biodegradable polymer is selected from the group consisting of polylactide (PLA), polyglycolide (PGA), poly(lactide-co-glycolide) (PLGA), monomethoxy(polyethylene glycol)-poly(lactide-co-glycolide) (mPEG-PLGA), polycaprolactone (PCL), poly ortho ester, polydioxanone, polyanhydride, polyhydroxyalkanoate, poly(etherurethane urea) (PEUU), poly(ether ester urethane)urea (PEEUU), (poly(ester carbonate urethane)urea (PECUU), (poly(carbonate urethane)urea (PCUU) and copolymers thereof.

In a more preferred embodiment the PLA, PGA, PLGA or PCL are used as further biodegradable polymer.

In another embodiment of the invention the further biodegradable polymer is a polysaccharide selected from the group consisting of alginate, chitosan, chitin, pectin, heparin sulfate and chondroitin sulfate. Utilization of polysaccharides can significantly enhance the wound healing properties of the wound care product.

According to the invention the collagen as part of the polymer layer is selected from the group consisting of types I, II and III collagens and mixtures thereof.

Soluble collagens of the types I, II and III collagen are prepared by limited enzymatic digestion of tissue enriched in such types of collagen and subsequent formation of a collagen-based solution (i.e. a soluble collagen dissolved in a suitable solvent, such as dilute hydrochloric acid, diluted acetic acid or the like).

Insoluble collagens can be obtained from the following typical sources: type I collagen: bovine, chicken and fish skin, bovine and chicken tendons and bovine and chicken bones including fetal tissues; type II collagen: bovine articular cartilage, nasal septum, sternal cartilage; and type III collagen: bovine and human aorta and skin.

In another embodiment of the invention the collagen as used for the polymer layer is a human recombinant collagen.

The term "human recombinant collagen" refers to collagen manufactured by culturing a non-human organism genetically modified to express at least one human gene encoding a collagen. The human recombinant collagen is suitably selected from the group consisting of collagen type I, type II, type III, type IV, type V, type VI, type VII, type VIII, type IX, type X, type XI, type XII, type XIII, type XIV, type XV, type XVI, type XVII, type XVIII, type XIX, type XX, type XXI, type XXII, type XXIII, type XXIV, type XXV, type XXVI, and type XXVII. The collagen can be collagen of one type free of any other type, or can be a mixture of different types of collagen. Suitably, the collagen comprises or consists essentially of collagens selected from the group consisting of type I collagen, type III collagen and mixtures thereof.

Human recombinant collagen may be provided by any suitable method known in the art. For example, the step of providing human recombinant collagen may comprise the following protocol described in U. S. Pat. No. 5, 962, 648, the entire content of which is incorporated herein by reference. Further recombinant processes are set forth in U.S. Pat. No. 5,593,859 and WO2004/078120, which are also incorporated herein by reference. Preferably, collagen is produced by culturing a cell which has been genetically modified to express at least one gene encoding the polypeptide comprising collagen and additional genes encoding the subunits of the post-translational modifying enzyme prolyl 4-hydroxylase, followed by the purification of the resultant collagen monomer therefrom. The recombinant collagen solution may be subsequently subjected to polymerization or cross-linking conditions to produce insoluble fibrous collagen.

Bovine collagen is a mixture of collagen type I (85%) and collagen type III (15%), a combination which is set by nature and cannot be varied. An advantage of recombinant collagen is that collagen type I and collagen type III are made independently of one another, and so any combination of type I and type III collagen can be made. The products according to the present invention may suitably comprise human collagen type I and human collagen type III in any ratio. For example, the products may comprise human collagen type I and human collagen type III in a ratio by weight of 100:0, 80:20, 60:40, 50:50, 40:60, 20:80 or 0:100, or anywhere in-between. Preferably, the ratio by weight of human collagen type I:human collagen type III is greater than about 50:50, and preferably it is greater than about 70:30, for example about 80:20. Suitably, the type I human recombinant collagen makes up at least about 75% by weight of the total human recombinant collagens in the material.

According to the invention the polymer layer comprises the collagen and the further biodegradable polymer in different forms such as:
(a) a biodegradable polymer/collagen blended fiber, which is preferably a nanofiber;
(b) a biodegradable polymer fiber coated with collagen;
(c) a collagen fiber coated with a biodegradable polymer; or
(d) a mixture of biodegradable polymer fibers and collagen fibers.

According to the invention a blended fiber is defined as a fiber that is composed of a mixture comprising at least one biodegradable polymer and at least one collagen. This fiber can be produced by preparing a solution comprising collagen and biodegradable polymers (or monomers or oligomers thereof) and a subsequent spinning process.

In a preferred embodiment, the fiber consisting of at least one further biodegradable polymer is coated with collagen.

The collagen coating can also be given as a core fiber of the further biodegradable polymer surrounded by an outer sheath of collagen.

Hereby, it is preferred that the collagen is not crosslinked after coating since it has been found out that residuals of the chemical cross-linking process remain in the device which might impair the wound healing process.

In a preferred embodiment of the invention the multi-layered wound care product has a moisture vapor transmission rate (MVTR) as measured according DIN ISO 13726-2 of more than 500 g/m²/24h, preferably of more than 750 g/m²/24h, and preferably of more than 1000 g/m²/24h. The parameters are a target for the test MVTR upright. And MVTR inverted is >20.000 g/m²/24h. For a wet product or hydrogel the DIN ISO 13726-2 should be adapted to measure only the vapor transmission rate and not a combination of dry loss and vapour transmission rate.

In a preferred embodiment of the invention the ECM layer represents the bottom layer of the multilayer of the invention and therefore is in contact to the wound, which is preferably located on the skin.

The multi-layered wound care product of the invention is particularly useful in the treatment of acute wounds, burn wounds, chronic wounds, and/or surgical wounds. The wound care product of the present invention may further be used in plastic surgery as well as for tissue engineering.

As such it can be used for the treatment of burns, partial and full-thickness wounds, pressure ulcers, venous ulcers, arterial ulcers, diabetic ulcers, chronic vascular ulcers, draining wounds, tunneled or undermined wounds, surgical wounds such as donor sites/grafts, post-Mohs surgery, post-laser surgery, podiatric dehiscence, and wound dehiscence, trauma wounds such as abrasions, lacerations, first, second, or third degree burns, and skin tears.

In the field of tissue engineering the wound care product of the invention can be used for remodelling of soft tissue, bone, cartilage, ligaments and tendons or dental applications.

For the medical use, it is required that the wound care product of the invention is provided in sterile form. This can be achieved by packaging the sterile product in a bacterial tight material with a marking on the packing that the product is sterilized. Bacterial tight materials are well known to the person skilled in art.

For preparing the polymer layer of multi-layered wound care of the invention the skilled person can use the various manufacturing methods as established for polymeric structures. For example the polymer layer can be produced by one or more methods selected from the group consisting of solvent-casting particulate-leaching, gas foaming, melt molding, freeze drying, rapid prototyping, solid freeform fabrication, electrohydrodynamic process (EHD), EHD-direct-jet process (EHD-JD), EHD direct writing, electrospinning and 3D printing.

In a further aspect the invention provides a method of preparing a multilayered wound care product comprising the following steps:
(a) provision of an ECM layer
(b) bringing the ECM layer in contact with a dispersion comprising collagen and at least one biodegradable polymer;
(c) freeze-drying the product of step (b).

In another aspect the invention provides a method of preparing a multilayered wound care product comprising the following steps:
(a) provision of a layer of foamed material, being preferably a sheet of PU foam;
(b) bringing said layer of foamed material in contact with a dispersion comprising collagen and at least one biodegradable polymer;
(c) drying or freeze-drying the product of step (b);
(d) coating the polymer layer coated-site of the product of step (c) with ECM by contacting it with a ECM-containing dispersion or an ECM sheet.
(e) drying or freeze-drying the product of step (d).

### EXAMPLES

### 1. Preparation of wound care product comprising an ECM layer and a collagen layer with a biodegradable polymer as connected by freeze-drying

ECM is prepared from animal material (e.g. skin) and laid on the bottom of culture dish which is then filled with 1-2% aqueous dispersion. The dispersion contains 0,5-2% Collagen, preferrably 1% Collagen, and 0,5-2% dispersed material (preferred 1%) of another biodegradable polymer like polylactide (PLA), polyglycolide (PGA), poly(lactide-co-glycolide) (PLGA), monomethoxy(polyethylene glycol)-poly(lactide-co-glycolide) (mPEG-PLGA) or polycaprolactone (PCL). The further biodegradable material can have an strong influence to the biochemical properties during wound healing. (e.g.polylactide increase the resorption time of the product and create an acetic wound environment during the resorption to lactic acid). The dish is frozen and subjected to freeze-drying.

As a result a freeze-dried composite composed of an ECM layer and a collagen/biodegradable polymer foam is given.

### 2. Preparation of an ECM layer/Collagen with a further biodegradable polymer with polyurethane film

The PU film has the role of regulating the water vapour permeation. On a freeze-dried ECM layer a polyurethane film is attached, e.g. by use of an acrylate adhesive, whereby the resulting acrylate layer can be regarded as intermediate layer in the context of the invention. Alternatively the two layers can be connected by freeze-drying. Hereto a thin layer of PU foam is used which has closed pores on one of its sides (e.g. Suprasorb M, Lohmann and Rauscher, Neuwied, Germany). The ECM dispersion is applied onto the open-pored side. An additional dispersion can be added on top. (To prevent a mixture a thickener can be added to the ECM dispersion e.g. hydroxyethylcellulose). The dispersion contains 0,5-2% Collagen, preferrably 1% Collagen and 0,5-2% dispersed material (preferred 1%) of a further biodegradable polymer like polylactide (PLA), polyglycolide (PGA), poly(lactide-co-glycolide) (PLGA), monomethoxy(polyethylene glycol)-poly(lactide-co-glycolide) (mPEG-PLGA) or polycaprolactone (PCL). The further biodegradable material can have a strong influence to the biochemical properties during wound healing. (e.g.polylactide increase the resorption time of the product and create an acetic wound environment during the resorption to lactic acid). The composition is frozen and freeze dried resulting in a wound care product with an intermediate layer. (As a result a freeze-dried composite composed of an ECM layer and a collagen/biodegradable polymer foam with a water steam regulation PU foam is given.

### 3. Preparation of a polyurethane foam (e.g. Cutimed Siltec, BSN Medical) with a collagen + other biodegradable polymer core and additional with a ECM wound contact layer

On the foamed site (wound contact layer) of a foam-containing product a collagen dispersion with another biodegradable polymer is given and allowed to soak into the polyurethane foam. The dispersion in produced according to example 2. The PU foam-collagen/biodegradable polymer mixture is dried at moderate temperatures (not exceeding 40°C) or alternatively freeze-dried. As a result a (freeze)dried composite of collagen/biodegradable polymer and PU foam is generated. On the wound contact layer of the PU foam with the collagen biodegradable polymer core a thin 0,5-3 mm preferred 1 mm layer of a ECM dispersion is coated with typical methods. The ECM dispersion is prepared according to prior art methods and can be supplemented with gel forming agents such as hydroxyethyl cellulose. The ECM material is preferably isolated from animal sources such as e.g. from skin. The PU foam-ECM mixture is dried at moderate temperatures (not exceeding 40°C) or alternatively freeze-dried. As a result a (freeze)dried composite of ECM and PU foam is generated. Notably, the PU foam can be equipped with a PU film on side facing away from the wound in order to have a defined MVTR value. The PU foam product can include further layers such as an adhesive layer or a layer of superabsorbent material, preferably between PU foam and PU film.

The reason for using the collagen/ biopolymer core and the ECM contact layer are the higher cost for an ECM material. The ECM has direct contact with the wound and can influence biochemical the wound environment. The collagen/biodegradable polymer core in the PU foam has additional an effect to the absorbed exudate like protease binding or to regulate the pH value of the wound environment if polylactide is used.

### 4. Preparation of a wound care product by adhesively connecting an ECM layer and a collagen layer

Hereby a freeze-dried ECM layer is attached to freeze-dried collagen with an additional biodegradable polymer layer by use of an adhesive. The two layers can be connected by a biodegradable film, preferably made from PLA, PGA, PLGA or PCL, which is equipped on both sides of the film with an acrylate glue. Alternatively, a hot melt adhesive can be used. This material is preferred when a reduced biodegradability is desired. In a third strategy, the ECM layer is coated with a collagen dispersion, the collagen layer is superimposed and the complete sandwich-structure is frozen and freeze-dried.

### 5. Preparation of a wound care product comprising an ECM layer and mixture of collagen and a biodegradable polymer

ECM is prepared from animal material (e.g. skin) and laid on the bottom of culture dish which is then filled with 1-2% aqueous collagen dispersion and afterwards overlayed with an aqueous solution of a biodegradable polymer. The dish is frozen and subjected to freeze-drying. As a result a freeze-dried composite composed of an ECM bottom layer and an intermediate collagen layer and a biodegradable polymer layer (upper layer) is given.

### 6. Preparation of a wound care product with a PU membrane, biodegradable membrane and ECM layer

Gelatine or collagen fibers are applied onto an ECM membrane. Collagen/gelatine fibers can be produced according prior art and can be obtained commercially, e.g. from Freudenberg SE (Weinheim, Germany). Onto this composite an aqueous solution of a biodegradable polymer is given which can be further supplemented by ECM particles. Finally a PU foam membrane such as Suprasorb M is added and the whole sandwich structure is frozen and freeze-dried.

### 7. Preparation of a collagen/ECM multilayered wound product (see Fig. 2D)

An ECM film/membrane (E1) such as Endoform (from Hollister Wound Care, Libertyville, IL, USA) is coated with a collagen dispersion and overlayed with a second ECM film/membrane. The components are connected by drying at moderate temperatures or by freeze-drying. Preferably, the two ECM layer are chosen in a way that they exhibit a different biochemical interaction with the wound site. The product can be equipped with a further collagen layer (S2) with a blended other biodegradable polymer, which is preferably a freeze-dried collagen/biodegradable polymer layer. The blended collagen/biodegradable polymer material can be produced with milling and mixing or with a dispersion (see 2). This collagen/other biodegradable polymer layer can be connected by use of an adhesive comprising a biodegradable polymer such as polylactide or a traditional adhesive. Alternatively, the collagen/ other biodegradable polymer can be added as highly viscous dispersion and cured by freeze-drying to result in a further connected collagen layer.

### Brief description of the drawings:

These and other aspects of the invention will be apparent from and elucidated with reference to the embodiments described hereinafter.

The invention will now be described, by way of example, based on embodiments with reference to the accompanying drawings.

In the drawings:
Fig. 1 shows a principal sketch of the wound care product according to a first embodiment in cross section (**A**) or in a perspective view (**B**), whereby (S) shows the polymer layer comprising collagen and at least one further biodegradable polymer and (E) denotes to the ECM layer attached thereto.
Figs. 2A to 2D show cross sections through different embodiments of a wound care product according to the present invention.

In the Figures, like numbers refer to like objects throughout. Objects in the Figures are not necessarily drawn to scale.

### Detailed description of embodiments:

Various embodiments of the invention will now be described by means of the Figures.

Fig. 1 shows a principal sketch of the wound care product according to a first embodiment in cross section (**A**) or in a perspective view (**B**) comprising of a polymer layer (S) and an ECM layer (E). The wound care product is shown in a cross section/perspective view and may be applied as such to a skin wound. To this, the wound care product has a surface that will face the wound when being applied and will come in direct contact with the wound, and has an opposite surface that will face away from the wound and will thus initially not contact the wound when applied. In the embodiment depicted in Fig. 1B, the lower layer provides the surface of the wound care product that will be contacting the wound when the wound care product is applied to a wound. The upper layer of this embodiment, as depicted in Fig. 1B, will face the opposite side and will thus initially not contact the wound when applied to the wound in this orientation. In the embodiment depicted in Fig. 1B, the upper layer may be considered a polymer layer (S) and the lower layer may be considered an ECM coating (E) that has been applied to the polymer layer. However, the upper layer may as well be considered a polymer coating (S) that has been applied to an ECM layer (E). Various ways of how to obtain the embodiment depicted in Fig. 1B will be described below. In use, i.e. when the wound care product is applied to a wound, for example a skin wound, the ECM coating or layer of the embodiment of Fig. 1 B will come in direct contact with the wound, whereas the polymer layer or coating will initially not be in contact with the wound.

However, once applied, the ECM material of the ECM layer will be absorbed or taken up and biodegraded by cells that start populating the wound from the edges of the wound as well as from cells from the circulating blood during the wound healing process. Accordingly, during the healing process, the ECM material of the ECM layer will be degraded, and the cells healing the wound as well as the wound itself will then also come in direct contact with the polymer layer.

Fig. 2A shows a cross-section taken along line I-I of the embodiment of Fig. 1. This cross-section further demonstrates the at least two distinct layers of this wound care product: a polymer layer (S), and an ECM layer (E). As can be seen in the embodiment shown in Fig. 2A, these layers of the wound care product are in direct contact with each other. As is further evident, there is no intermingling between both layers, but both layers are demarcated in the displayed cross-section by a single line of contact (corresponding to an area of contact in the entire product).

In a further embodiment depicted in Fig. 2B, the polymer layer (S) and the ECM layer (E) may be separated by an additional intermediate layer (IL) that is located between both layers. According to this embodiment of the wound care product, there is no direct contact between the polymer layer (S) and the ECM layer (E). Referring now to the embodiment depicted in Fig. 2C, the wound care product according to the present invention may have more than two layers, such as two ECM layers (E1, E2), one on either side of a polymer layer (S). Preferably, the ECM layers (E1, E2) of this embodiment are obtained by adding a coating of ECM material in the form of a powder to a polymer layer (S). As shown in Fig. 2D, two ECM layers (E1 and E2) have been applied to a polymer layer (S1), and a further polymer layer (S2) has been additionally applied to the second ECM layer (E2). Accordingly, the wound care product of this embodiment includes alternating polymer and ECM layers.

While the invention has been illustrated and described in detail in the drawings and the foregoing description, such illustration and description are to be considered illustrative or exemplary and not restrictive.

From reading the present disclosure, other modifications will be apparent to persons skilled in the art. Such modifications may involve other features which are already known in the art and which may be used instead of or in addition to features already described herein.

Variations to the disclosed embodiments can be understood and effected by those skilled in the art, from a study of the drawings, the disclosure and the appended claims. In the claims, the word "comprising" does not exclude other elements or steps, and the indefinite article "a" or "an" does not exclude a plurality of elements or steps. The mere fact that certain measures are recited in mutually different dependent claims does not indicate that a combination of these measures cannot be used to advantage.

Any reference signs in the claims should not be construed as limiting the scope thereof.

### Definitions

The term "biodegradable polymer" as used in the context of the present invention refers to a polymer that is degraded under physiological conditions. In some embodiments, the biodegradable polymer is a polymer that is broken down by cellular machinery. In some embodiments, the biodegradable polymer is a polymer that is broken down by chemical processes. Due to the expression "further biodegradable polymer" it is clarified, that within the polymer matrix of the invention this polymer has be present in addition to the biodegradable polymer collagen. As such it is excluded that the further biodegradable polymer of the invention could be collagen.

The further biodegradable polymer of the invention is preferably a biocompatible polymer. As used in the context of the invention, the term "biocompatible" relates to a polymer that is substantially non-toxic in an *in vivo* environment, and is not substantially rejected by a recipient's physiological system.

The term "decellularized ECM sheet" as used herein refers to a tissue from which a substantial amount of cellular and nucleic acid content has been removed leaving behind a complex interstitial structure of ECM that can be used as a scaffold for wound healing or tissue regeneration. In a preferred embodiment said term refers to an ECM material that has been cleaned, disinfected, sterilized and optionally cross-linked.

The term "hydrogel" refers to a three-dimensional (3D) crosslinked network of hydrophilic polymers that swell in water. In some embodiments, water can penetrate in between the polymer chains of the polymer network, subsequently causing swelling and the fomlation of a hydrogel. In general, hydrogels are superabsorbent. For example, in some embodiments, hydrogels can contain 50% or more, 60% or more, 70% or more, 80% or more, 90% or more, 95% or more, or 98% or more by weight of water. In some embodiments, further components such as proteins can be encapsulated within hydrogels. Typically, these further components are encapsulated within hydrogels through mixing a cell suspension and/or ECM molecules with a precursor solution (i.e., a solution comprising a polymer suitable for hydrogel formation) and crosslinking the resulting network using any available means for crosslinking. In some embodiments, a plurality of hydrogels can be assembled together to form a hydrogel assembly.

### List of reference numerals:

- E, E1, E2: ECM-layer
- S, S1, S2: Polymer layer
- IL: Intermediate layer

## Claims

1. A multilayered wound care product comprising:
(a) at least one extracellular matrix (ECM) layer;
(b) at least one polymer layer comprising collagen and at least one further biodegradable polymer; and optionally
(c) a covering layer.

2. The multilayered wound care product according to claim 1, wherein the ECM layer consists of a particulate ECM or sheet-like ECM, which is preferably decellularized.

3. The multilayered wound care product according to claim 2, wherein the ECM layer consists of an intact ECM sheet with a thickness between 50 and 250 µm, preferably between 75 and 150 µm and more preferably between 80 and 120 µm.

4. The multilayered wound care product according claim 1 to 3, wherein the ECM layer is prepared *in vitro* from cultured chondrocytes, fibroblasts, keratinocytes or epithelial cells; or prepared *in vivo* from intestinal tissue, bladders, liver, spleen, stomach, lymph nodes or skin and preferably from human cadaver skin, porcine urinary bladder submucosa (UBS), porcine urinary bladder matrix (UBM), or porcine small intestine submucosa (SIS).

5. The multilayered wound care product according to any of the above claims, wherein the at least one polymer layer is directly attached to the at least one ECM layer.

6. The multilayered wound care product according to any of the above claims, wherein the at least one polymer layer is a 3D-scaffold.

7. The multilayered wound care product according to any of the above claims, wherein the at least one polymer layer comprises one or more of the following:
(a) a biodegradable polymer/collagen blended fiber, which is preferably a nanofiber;
(b) a biodegradable polymer fiber coated with collagen;
(c) a collagen fiber coated with a biodegradable polymer; or
(d) a mixture of biodegradable polymer fibers and collagen fibers.

8. The multilayered wound care product according to any of the above claims, wherein the further biodegradable polymer is selected from the group consisting of polylactide (PLA), polyglycolide (PGA), poly(lactide-co-glycolide) (PLGA), monomethoxy(polyethylene glycol)-poly(lactide-co-glycolide) (mPEG-PLGA), polycaprolactone (PCL), poly ortho ester, polydioxanone, polyanhydride, polyhydroxyalkanoate, and copolymers thereof.

9. The multilayered wound care product according to any of claim 1 to 7, wherein the further biodegradable polymer is a polysaccharide selected from the group consisting of alginate, chitosan, chitin, pectin, heparin sulfate and chondroitin sulfate.

10. The multi-layered wound care product according to any of the above claims, wherein the multi-layered wound care product has a moisture vapor transmission rate (MVTR) as measured according to DIN-ISO 13726-2 of more than 500 g/m²/24h, preferably of more than 750 g/m²/24h, and preferably of more than 1000 g/m²/24h.

11. The multi-layered wound care product according to any of the above claims, wherein the ECM layer represents the skin contact layer.

12. The multi-layered wound care product according to any of the above claims for use in the treatment of chronic wounds, comprising burns, partial and full-thickness wounds, pressure ulcers, venous ulcers, arterial ulcers, diabetic ulcers, chronic vascular ulcers, draining wounds, tunnelled or undermined wounds, surgical wounds such as donor sites/grafts, post-Mohs surgery, post-laser surgery, podiatric dehiscence, and wound dehiscence, trauma wounds such as abrasions, lacerations, second-degree burns, and skin tears.

13. The multi-layered wound care product to any of the above claims, wherein the polymer layer is produced by one or more methods selected from the group consisting of solvent-casting particulate-leaching, gas foaming, melt molding, freeze drying, rapid prototyping, solid freeform fabrication, electrohydrodynamic process (EHD), EHD-direct-jet process (EHD-JD), EHD direct writing, electrospinning and 3D printing.

14. A method of preparing a multilayered wound care product according to any of claims 1 to 11 comprising the steps of:
(a) provision of an ECM layer;
(b) bringing the ECM layer in contact with a dispersion comprising collagen and at least one biodegradable polymer;
(c) freeze-drying the product of step (b).

15. A method of preparing a multilayered wound care product to any of claims 1 to 11 comprising the following steps:
(a) provision of a layer of foamed material, being preferably a sheet of PU foam;
(b) bringing said layer of foamed material in contact with a dispersion comprising collagen and at least one biodegradable polymer;
(c) drying or freeze-drying the product of step (b);
(d) coating the polymer layer coated-site of the product of step (c) with ECM by contacting it with a ECM-containing dispersion or an ECM sheet;.
(e) drying or freeze-drying the product of step (d).
